# EUROPEAN PATENT APPLICATION

(11) **EP 2 065 034 A2**
(43) Date of publication of application: **03.06.2009**
(21) Application number: 08104379.6
(22) Date of filing: 11.06.2008
(51) Int. Cl.: A61K 9/16, A61K 9/50

(54) **Enteric-coated glucosinolates and beta-thioglucosidases**

(30) Priority: 12.06.2007 US 761843
(71) Applicant: Kraft Foods Holdings, Inc., Northfield, IL 60093 (US)
(72) Inventor: West, Leslie George, Winnetka, IL 60093 (US); Windsor, Nicole Lee, Chicago, IL 60613 (US); Gaonkar, Anilkumar Ganapati, Buffalo Grove, IL 60089 (US); Matusheski, Nathan V., Gurnee, IL 60031 (US); Kim, Nam-Cheol, Deerfield, IL 60015 (US); Ludwig, Cathy Jean, Grayslake, IL 60030 (US); Lawrence, Leslie Lewis, Glenview, IL 60025 (US)
(74) Representative: Smaggasgale, Gillian Helen

(57) **Abstract**

The present invention relates to a particulate composition comprising enteric-coated glucosinolate and beta-thioglucosidase particles. The present invention further provides a method of converting glucosinolate to isothiocyanate in the small intestine comprising orally administering to a subject an enteric-coated chemoprotectant precursor composition comprising enteric-coated glucosinolate and beta-thioglucosidase particles. In another aspect, uncoated glucosinolate and beta-thioglucosidase particles may be provided in an enteric-coated capsule. Preferably, the glucosinolate is glucoraphanin and the beta-thioglucosidase is myrosinase. The enteric coating targets the compound for release in the small intestine where beta-thiaglucosidase enzyme converts glucosinolate to chemoprotectant isothiocyanate.

## Description

The present invention relates to a composition comprising enteric-coated glucosinolate and enteric-coated beta-thioglucosidase enzyme particles. The present invention further provides a method of converting glucosinolate to isothiocyanate in the small intestine comprising orally administering to a subject an enteric-coated chemoprotectant precursor composition comprising enteric-coated glucosinolate and beta-thioglucosidase particles. In another aspect, uncoated glucosinolate and beta-thioglucosidase particles may be provided in an enteric-coated capsule. The enteric coating targets the chemoprotectant precursor composition for release in the small intestine where beta-thioglucosidase converts the glucosinolate to chemoprotectant isothiocyanate. More specifically, the chemoprotectant precursor composition comprises enteric-coated glucoraphanin and its conversion enzyme myrosinase.

### BACKGROUND

It is generally agreed that diet plays a large role in controlling the risk of developing cancers and other diseases and conditions, such as ulcers and cardiovascular disease, and that increased consumption of fruits and vegetables may reduce cancer incidences in humans. The presence of certain minor chemical components in plants may provide protection mechanisms when delivered to mammalian cells. Moreover, providing pharmaceuticals, nutritional supplements, or foods fortified or supplemented with cancer-fighting chemical components derived from plants may provide additional health benefits. An important trend in the U.S. food industry is to promote health conscious food products.

Cruciferous vegetables contain phytochemicai precursors to potent chemoprotectants, especially the precursor glucoraphanin (which is also known as sulforaphane glucosinolate or 4-methylsulfinylbutyl glucosinolate) and its associated enzymatic conversion product sulforaphane, that appear to trigger carcinogen detoxification mechanisms when delivered to mammalian cells. Glucosinolates are found in dicotyledenous plants and most commonly in the Brassicaceae (Cruciferae) family. Glucosinolates are sulfur-containing compounds of the general structure: 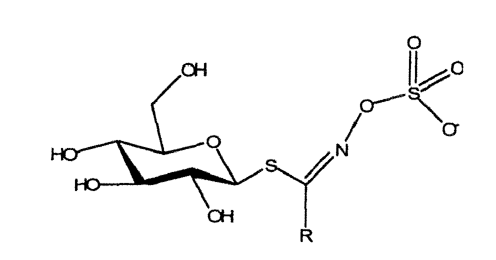 Glucosinolates includes an R-group derived from amino acids and a thioglucosidic link to a sulphonated oxime. The thioglucosidic bonds of the glucosinolates are hydrolyzed by beta-thioglucosidases into unstable glucosinolate aglycones, which undergo spontaneous rearrangement into isothiocyanates, such as sulforaphane.

In addition to reducing the risk of certain cancers, glucoraphanin, through its bioactive conversion product sulforaphane, has recently been shown effective in destroying organisms responsible for causing the majority of stomach ulcers and may provide novel approaches for reducing the risk of developing cardiovascular and ocular diseases. Efforts are being made to gain approval for making label claims on food products either naturally high in these agents or for foods containing added crucifer chemoprotectants. Products containing chemoprotectant additives, although without such label claims, are already on the market.

Even though isothiocyanates, particularly sulforaphane, are increasingly recognized as important wellness-enablers, isothiocyanates typically are not used in food products or supplements due to their extremely pungent taste. Isothiocyanates also have high chemical reactivity, which makes isothiocyanates poor candidates for encapsulation.

To overcome the problems inherent in isothiocyanates, glucosinolates are generally provided in food products and health supplements instead. Administration of glucosinolates without the converting enzymes still results in the formation of isothiocyanates by gut microflora but at substantially reduced levels. While the dosage of glucosinolates may be increased to reduce this problem, there is considerable cost in doing so. It is also believed that isothiocyanates are not well absorbed in the stomach but instead are more readily absorbed in the small intestine. Additionally, the formation of isothiocyanates in the gut can lead to loss of isothiocyanates due to the low pH-favored formation of non-health promoting derivatives, especially nitriles.

Therefore, there remains a need for more optimal formulations designed to deliver isothiocyanates to the small intestine where the isothiocyanates can be readily absorbed. The present invention fulfills these, as well as other needs, as will be apparent from the following description of embodiments of the present invention.

### SUMMARY

The present invention provides enteric-coated glucosinolate and beta-thioglucosidase enzyme particles which are prepared separately or in combination by conventional methods. Enteric-coated glucosinolates and beta-thioglucosidase enzyme particles may be prepared together under conditions designed to substantially reduce the rate of reaction between the glucosinolate and beta-thioglucosidase enzyme. In another aspect, uncoated glucosinolate and beta-thioglucosidase particles may be incorporated into enteric coated capsules, tablets, or the like. Upon digestion, the enteric coating remains intact while passing through the stomach and only dissolves in the small intestine to release the beta-thioglucosidase and glucosinolate particles. The beta-thioglucosidase enzyme converts glucosinolates into chemoprotectant isothiocyanates within the small intestine. The enteric coating allows the glucosinolates and their conversion enzymes to arrive intact in the small intestine where absorption of isothiocyanates is believed to be most efficient. Preferably, the glucosinolates are glucoraphanin and the beta-thioglucosidase enzymes are myrosinase, which converts glucoraphanin into sulforaphane, a potent chemoprotectant.

Generally, the method of coating the glucosinolate and beta-thioglucosidase particles can be carried out by any means known in the art. Thus, for example, the following method can be used: (1) preparing a homogenous mixture containing active agent, coating excipient, and optionally diluent; (2) adding liquid, such as water, propylene glycol, glycerol, sorbitol, and mixtures thereof, to the dry mixture to form a wet mass suitable for wet extrusion; (3) granulating and extruding the wet mass to form extrudate; (4) forming particles from the extrudate; and (5) drying the particles. In a preferred aspect, the particles in step (4) are formed by spheronization. In a particularly preferred aspect, the particles formed in step (4) are substantially-spherical in shape.

The particles thus formed can then be coated with an enteric coating. Enteric coatings include any barrier known in the art that is applied to oral medications, food supplements, or the like that prevents the release of the active agent before it reaches the small intestine. Enteric coatings prevent the destruction of the active agent by the acidic environment of the stomach. Alternatively, uncoated particles may be filled into capsules, which are then coated with an enteric coating. The enteric coating on the particles or capsules provides for the release of the glucosinolate and beta-thioglucosidase enzyme particles in the small intestine where beta-thioglucosidase converts glucosinolates into isothiocyanates.

Suitable enteric coatings include shellac, methacrylic acid copolymers and their derivatives, cellulose acetate, styrol maleic acid copolymers, polymethacrylic acid/acrylic acid copolymer, hydroxylpropyl methyl cellulose phthalate, polyvinyl acetate phthalate, hydroxyethyl ethyl cellulose phthalate, hydroxypropyl methyl cellulose acetate succinate, cellulose acetate tetrahydrophthalate, acrylic resin, timellitate, zein, calcium alginate, fatty acids, fats, and combinations thereof, among others. Examples of suitable commercially available enteric coatings include, but are not limited to, MARCOAT® 125 from Emerson Resources, Inc. (Norristown, PA), EUDRAGIT® from Degussa, or Cellulose Acetate Phthalate, NF ("CAP") from Eastman Chemical Co. (Kingston, TN), or the like.

In another aspect, food products and pharmaceuticals are provided that include the enteric-coated glucosinolate and beta-thioglucosidase particles. The enteric-coated glucosinolate and beta-thioglucosidase particles of the invention may be used in a wide variety of food applications, pharmaceuticals, or the like. The enteric-coated glucosinoiate and beta-thioglucosidase particles may be incorporated directly or may be further processed, as desired, before incorporation into food products or pharmaceuticals. Food products into which the enteric-coated glucosinolate and beta-thioglucosidase particles may be incorporated include food supplements, nutrition bars, cereals, biscuits, drinks, shakes, pills, tablets, powdered beverage mixes, and the like. Supplements include dietary supplements, nutritional supplements, herbal supplements, and the like.

The present invention further provides a method of converting glucosinolate to isothiocyanate in the small intestine comprising orally administering to a subject an enteric-coated chemoprotectant precursor composition comprising enteric-coated glucosinolate and beta-thioglucosidase particles. In another aspect, uncoated glucosinolate and beta-thioglucosidase particles may be provided in an enteric-coated capsule. The enteric coating targets the chemoprotectant precursor composition for release in the small intestine where beta-thioglucosidase converts the glucosinolate to chemoprotectant isothiocyanate.

### BRIEF DESCRIPTION OF THE FIGURES

FIG. 1 provides the principal reaction for conversion of glucoraphanin to sulforaphane.

FIG. 2 provides a flowchart illustrating one embodiment of the process of the invention.

FIG. 3 provides a flowchart illustrating another embodiment of the process of the invention.

FIG. 4 provides a flowchart illustrating another embodiment of the process of the invention.

FIG. 5 illustrates several embodiments of coated particles. Part A illustrates a single particle, such as comprising either glucosinolate or beta-thioglucosidase, is coated with a coating. Part B illustrates a plurality of particles of a single type, such as comprising either glucosinolate or beta-thioglucosidase, coated together with a coating. Part C illustrates a plurality of particles of more than one type, comprising both glucosinolate and beta-thioglucosidase, coated with a layer of coating.

### DETAILED DESCRIPTION

The present invention is directed to enteric-coated glucosinolate and beta-thioglucosidase enzyme particles. Generally, the glucosinolate may be glucoraphanin, glucoraphenin, glucoerucin, the like, or a combination thereof. In a preferable aspect, the glucosinolate is glucoraphanin and the beta-thioglucosidase enzyme is myrosinase, which converts glucoraphanin to sulforaphane, a chemoprotectant. The enteric-coated glucosinolates and beta-thioglucosidase enzyme particles can be produced separately or in combination by conventional methods. Alternatively, uncoated glucosinolate and beta-thioglucosidase particles can be combined and incorporated in enteric-coated capsules.

The present invention is further directed to a method of converting glucosinolate to isothiocyanate in the small intestine comprising orally administering to a subject an enteric-coated chemoprotectant precursor composition comprising enteric-coated glucosinolate particles and enteric-coated beta-thioglucosidase particles. Alternatively, the glucosinolate particles and beta-thioglucosidase particles may be coated or uncoated and provided in an enteric-coated capsule.

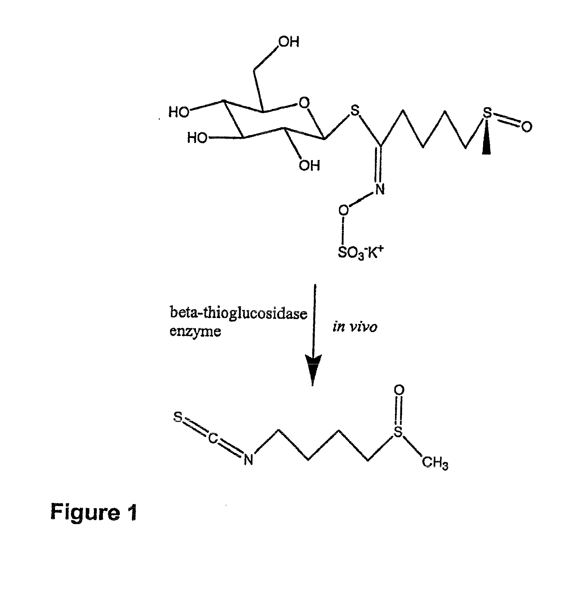

A significant advantage of an enteric-coated formulation comprising glucosinolate and beta-thioglucosidase particles is that the enteric coating allows glucosinolates and the beta-thioglucosidases to arrive intact in the small intestine where absorption of isothiocyanates is believed to be most efficient. Upon delivery to the small intestine, the enzyme from the provided enteric-coated beta-thioglucosidase particles or the beta-thioglucosidase particles from the provided enteric-coated capsules are released and convert the provided glucosinolate particles into chemoprotectant isothiocyanates. Preferably, the glucosinolate is glucoraphanin and the beta-thioglucosidase enzyme is myrosinase, which converts glucoraphanin into sulforaphane, a potent chemoprotectant. The principal reaction is illustrated in FIG. 1.

As used herein, "chemoprotectants" and "chemoprotectant compounds" refer to agents of plant origin that are effective for reducing the susceptibility of mammals to the toxic and neoplastic effects of carcinogens. Chemoprotectant "precursors" refer to agents which give rise to chemoprotectants by enzymatic and/or chemical means. Talalay, P. et al., J. Nutr., 131 (11 Suppl.): 30275-30335 (2001). Examples of such chemoprotectant precursors include alkyl glucosinolates, such as glucoraphanin.

As used herein, "active agent" means glucosinolates, preferably glucoraphanin and/or beta-thioglucosidase enzyme, preferably myrosinase, or a mixture thereof.

As used herein, "effective amount" is an amount of active agent which provides the desired effect or benefit upon consumption. Generally, about 1 to about 100 mg of glucosinolate, preferably glucoraphanin, and about 1 to about 100 mg of beta-thioglucosidase, preferably myrosinase, per single serving of the food product or pharmaceutical composition would be considered to be effective.

Glucosinolates derived from crucifer seeds or sprouts are useful starting materials. Crucifer seeds and sprouts have been found to be an especially good source of chemoprotectant precursors. Crucifer seeds or sprouts which are especially useful include broccoli, kale, collard, curly kale, marrowstem kale, thousand head kale, Chinese kale, cauliflower, Portuguese kale, Brussels sprouts, kohlrabi, Jersey kale, savoy cabbage, collards, borecole, radish, and the like as well as mixtures thereof. In a very important aspect, crucifier seeds or seeds and sprouts of broccoli are utilized.

Glucosinolate extracts or isolates may generally be prepared by any means known in the art and can include the methods disclosed in co-pending U.S. Application Serial Nos. 11/617,934, filed December 29, 2006, and 11/199,752, filed August 9, 2005 and 11/761,883 filed 12 June 2007,
which are commonly assigned to the assignee herein and which are incorporated by reference as if reproduced in their entirety herein.

Generally, glucosinolate and beta-thioglucosidase particles are prepared by the method comprising: (1) preparing a homogenous mixture of active agent, coating excipient, optionally diluent, and optionally enzyme activator; (2) adding liquid, such as water, propylene glycol, glycerol, sorbitol, or mixtures thereof, preferably water, to the dry mixture to form a wet mass suitable for wet extrusion; (3) granulating and extruding the wet mass to form extrudate; (4) forming particles from the extrudate; and (5) drying the particles. Those of ordinary skill in the art will understand that there are several processes known in the art for forming particles from extrudate, particularly forming particles that are suitable for coating, such as fluidized bed coating, spinning disc, spray drying, and the like. The dried particles may then be coated with an enteric coating, or may be filled in a capsule that is then coated with a release modifying coating, preferably an enteric coating.

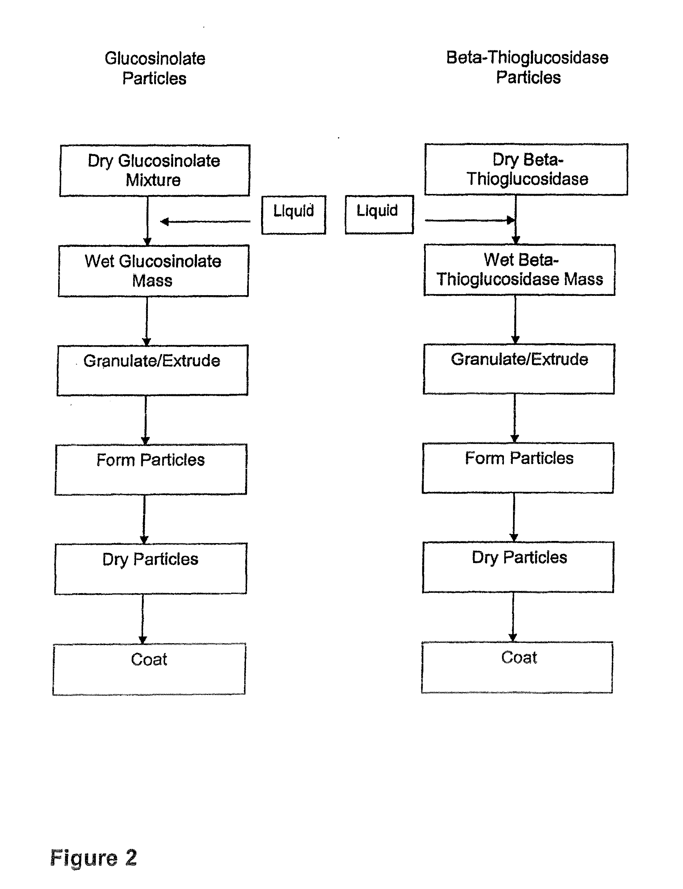

In one embodiment, as shown in FIG. 2, the glucosinolate and beta-thioglucosidase particles are produced and coated separately to prevent reaction between the glucosinolates and beta-thioglucosidase enzymes during processing. Once coated, the coated beta-thioglucosidase and glucosinolate particles may be mixed together, preferably in a ratio of about 1:100 to about 100:1, more preferably about 1:10 to about 10:1, although other ratios may be used if desired. One of skill in the art that the ratio used may depend on the relative purities of the beta-thioglucosidase and glucosinolate sources used. This ratio provides an enzyme to substrate ratio for the conversion reaction to occur at a sufficient rate while the enzyme and substrate reside in the small intestine.

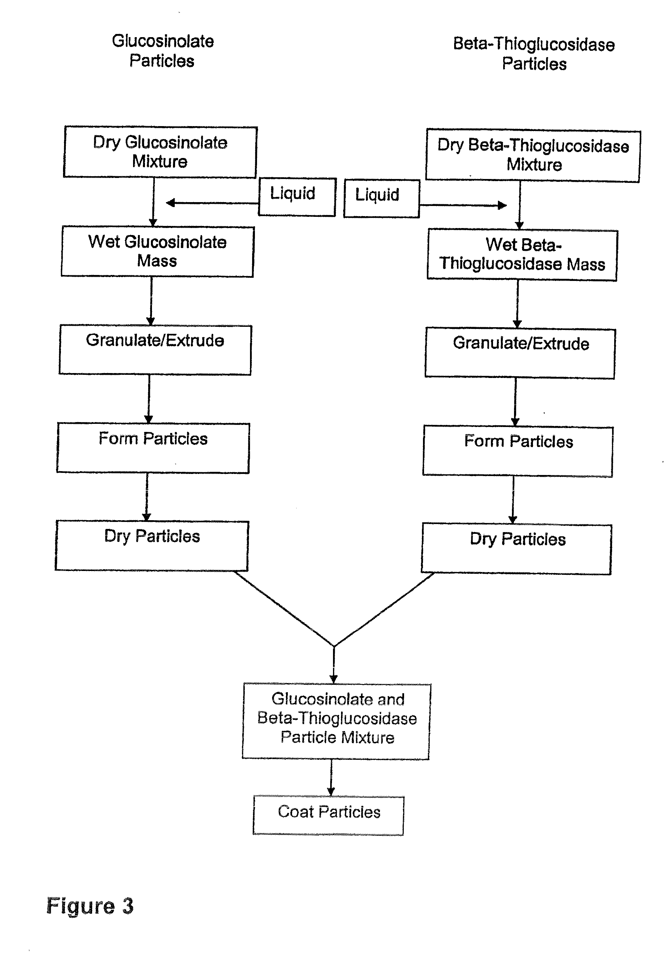

In another embodiment, as shown in FIG. 3, glucosinolate and beta-thioglucosidase particles can be produced separately and then combined to form a mixture after drying step (5) and prior to coating. Generally, the beta-thioglucosidase and glucosinolate particles are combined in a ratio of about 1:100 to about 100:1, preferably about 1:10 to about 10:1, although other ratios of beta-thioglucosidase and glucosinolate particles may be used if desired.

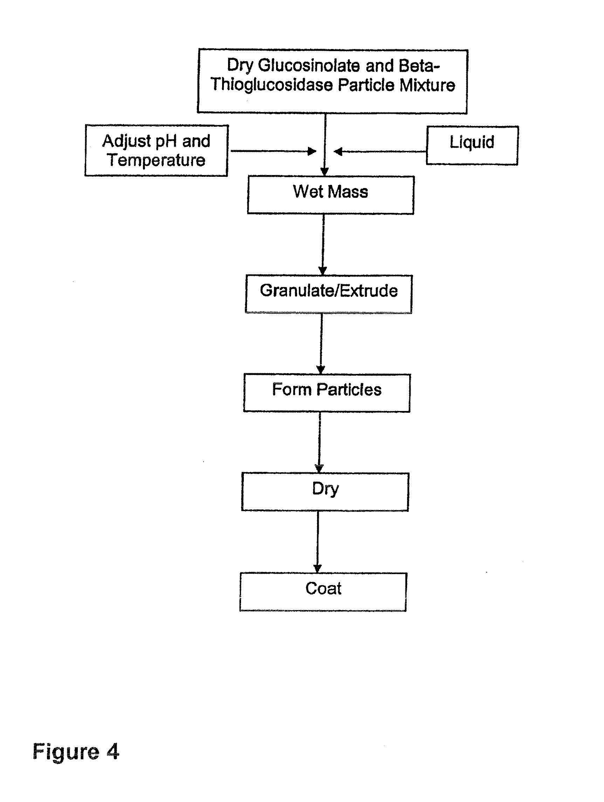

In another embodiment, as shown in FIG. 4, glucosinolate and beta-thioglucosidase particles can be produced together beginning at step (1) if processing conditions are adjusted and/or maintained to substantially reduce the likelihood of the beta-thioglucosidase enzymes catalyzing the conversion of glucosinolates into isothioglucosidases. In this aspect, glucosinolates and beta-thioglucosidases can be combined when forming the dry homogenous mixture of step (1) if, when adding liquid in step (2), the pH is adjusted to about 2 to about 3 and the temperature is adjusted to about 0 to about 15°C in order to substantially reduce the rate of conversion of glucosinolates to isothiocyanates. The temperature in this range should be maintained until the glucosinolate and beta-thioglucosidase particles are dried.

The active agents are glucosinolate, beta-thioglucosidase, or a mixture thereof. The active agents are generally in the form of a dried extract, isolate, purified isolate, semi-purified isolate, or the like. Preferably, the glucosinolate is glucoraphanin and the beta-thioglucosidase enzyme is myrosinase.

The coating excipient can comprise microcrystalline cellulose, lactose, oat fiber, carboxy methyl cellulose, derivatives thereof, the like, or mixtures thereof. Preferably, the coating excipient is a spheronizing agent. The spheronizing agent may be any spheronizing agent known in the art such as microcrystalline cellulose and its derivatives, oat fiber, carboxy methyl cellulose, derivatives thereof, the like, or mixtures thereof. More preferably, the spheronizing agent is microcrystalline cellulose, such as Microcrystalline Cellulose GP-1030 from FMC BioPolymer (Philadelphia, PA). The spheronizing agent provides plasticity to the mixture to enable particle formation and provides strength to the particles once formed.

The particles may optionally include diluent. The diluent may include any inert, food grade or pharmaceutically-acceptable substance, such as lactose, starch, dextrin, water, glycerol, sorbitol, propylene glycol, the like, or a mixture thereof. The diluent may also function as a binder. Lactose may serve as a coating excipient or a diluent. Preferably, the diluent is lactose.

Optionally, an enzyme activator may be included in the active agent mixture. The enzyme activator may comprise ascorbic acid and its derivatives. Preferably, the enzyme activator is ascorbic acid. Upon digestion and dissolution of the enteric coating, the enzyme activator increases the rate of reaction between beta-thioglucosidase and glucosinolate.

As described above, it may be desirable to prepare glucosinolate and beta-thioglucosidase particles separately, such as illustrated in FIGS. 2 and 3, or in combination, such as illustrated in FIG. 4.

Generally, to prepare an active agent mixture comprising beta-thioglucosidase, about 1 to about 50 percent beta-thioglucosidase is mixed with about 50 to about 99 percent composition comprising coating excipient and optionally diluent to form a dry beta-thioglucosidase mixture. Preferably, about 20 to about 30 percent beta-thloglucosidase is mixed with about 70 to about 80 percent composition comprising coating excipient and optionally diluent. In a particularly preferred aspect, the mixture also includes about 0.001 to about 10 percent enzyme activator, preferably ascorbic acid.

Generally, to prepare an active agent mixture comprising glucosinolate, about 1 to about 50 percent glucosinolate is mixed with about 50 to about 99 percent composition comprising coating excipient and optionally diluent to form a dry glucosinolate mixture. Preferably, about 20 to about 30 percent glucosinolate is mixed with about 70 to about 80 percent composition comprising coating excipient and optionally diluent.

Generally, to prepare an active agent mixture comprising both glucosinolate and beta-thioglucosidase, the mixture is prepared by combining about 0.5 to about 50 percent beta-thioglucosidase, about 0.5 to about 50 percent glucosinolate, and about 1 to about 99 percent composition comprising coating excipient and optionally diluent. Preferably, the mixture is prepared by combining about 0.5 to about 25 percent beta-thioglucosidase, about 0.5 to about 25 percent glucosinolate, and about 50 to about 99 percent composition comprising coating excipient and optionally diluent. More preferably, about 10 to about 15 percent beta-thioglucosidase is mixed with 10 to about 15 percent glucosinolate, and about 70 to about 80 percent composition comprising coating excipient and optionally diluent. In a particularly preferred aspect, the mixture also includes about 0.001 to about 10 percent enzyme activator, preferably ascorbic acid.

The ingredients of the active agent mixture can be combined in any convenient order. The resulting mixture is stirred, mixed, blended, or agitated by any convenient means until a homogenous dry mixture is formed.

The active agent mixture may also have introduced optional ingredients or components, such as, for example, nutrients, vitamins, colorants, nutraceutical additives, antioxidants, probiotics, prebiotics, sweetening agents, flavoring agents, processing agents, or the like so long as they do not adversely affect the processing or stability properties in a significant manner. Generally, these optional ingredients or components comprise about 0.1 to about 10 percent.

Preferably, the homogenous dry mixture is formed prior to wetting with a liquid, although the dry ingredients and liquid may be combined and mixed without first forming a homogenous dry mixture. Generally, a better and more homogenous mixture (dough) is formed when the homogenous dry mixture is formed prior to wetting. The dry mixture is wetted with a liquid, such as water, glycerol, propylene glycol, sorbitol, alcohol, the like, or mixtures thereof. Preferably, the liquid is water at a temperature of about 0 to about 15°C. The amount of liquid added to the dry mixture is the minimum amount required to prepare an extrudable mass. When spheronization is used to prepare the particles, the size of the particles produced during spheronization is largely determined by the amount of liquid added to produce the wet mass. The amount of liquid also affects the particle size distribution and plastic deformability characteristics of the particles. The particles must have sufficient plasticity to allow deformation during collisions but yet be strong enough to not break apart during spheronization (i.e., too much liquid causes the extruded mass to not break to form spherical particles while too little liquid causes the wet mass to crumble and break into a fine powder upon spheronizing).

Generally, the beta-thioglucosidase mixture is wetted with a liquid to a wet mass percentage of about 10 to about 50 percent, preferably about 20 to about 40 percent, to form an active agent wet mass.

Generally, the glucosinolate mixture is wetted with a liquid to a wet mass percentage of about 10 to about 50 percent, preferably about 20 to about 40 percent, to form an active agent wet mass.

Generally, the glucosinolate and beta-thioglucosidase mixture is wetted with a liquid to a wet mass percentage of about 10 to about 50 percent, preferably about 20 to about 40 percent, to form an active agent wet mass.

The active agent wet mass is then granulated and extruded using a conventional granulator, such as the MG-55 Single-Screw Multi-Granulator distributed by LCI Corporation (Charlotte, NC). Each active agent wet mass is separately fed through the hopper and granulated at a screw rotational speed of about 20 to about 100 rpm though the extruder fitted with the appropriate die (or screen) to form a cylindrical extrudate. Generally, the diameter of the extrudate determines size of the particles. Preferably, a 0.8mm/0.8T dome die is used to form the extrudate.

The extrudate is then formed into particles by any conventional method, such as by fluidized bet coating, spinning disc, spray drying, or the like. The particles so formed may be any shape, such as spherical, spheroidal, angular, tabular, irregular, ellipsoidal, discoidal, rod shaped, or the like, although it is preferable that the particles are substantially uniform in size and shape. Preferably, the particles are about 100 to about 1500 µm in diameter, more preferably about 200 to about 800 µm in diameter. In a particularly preferred aspect, the particles are formed by spheronization. Spheronization is a commonly used method for producing particles suitable for coating. Generally, spheronized particles have a uniform size and shape and have a low surface area to volume ratio. The spheronized particles may be spheres, spheroids, rounded rods, or the like, although preferably the spheronized particles are sphere-shaped. Spheronized particles also have a smooth surface that is ideal for coating. Spheronized particles can be uniformly coated with a minimum amount of coating material. Such uniform coating assists in providing uniform release among the particles in the small intestine. The extrudate is processed in conventional spheronizing equipment, e.g., Marumerizer model QJ-230T, manufactured by Fuji Paudal (Osaka, Japan) and distributed by LCI Corporation (Charlotte, NC). The spheronizing equipment breaks the cylindrical extrudate into small particles and tends to round the particles. The speed used for the rotating friction disk in the spheronizing equipment depends on the desired particle size of the particles. Generally, the production of smaller particles requires higher speeds than the production of larger particles. The extrudates are separately spheronized at a disk speed of about 500 to about 3000 rpm for about 1 to about 5 minutes or until the particles are substantially uniform in both size and shape. After about 1 to about 5 minutes, the wet particles are discharged into an appropriate receptacle. The particles thus-formed are ideally suited for coating because of the substantially smooth, uniform shape.

The wet particles are collected and dried by any conventional means known in the art, such as by fluid bed drying, drum drying, freeze drying, tray drying, conventional oven, or the like. Preferably, the wet particles are dried by fluidizing. For example, the particles can be fluidized at about 35°C to about 70°C for about 10 to about 60 minutes in a conventional fluid bed dryer, such as a Mini-Glatt (distributed by Glatt Air Techniques, Inc., Ramsey, NJ).

The particles thus-formed can then be coated to modify the release properties of the particles, such as with a delayed release coating, sustained release coating, controlled release coating, targeted release coating, enteric coating, and the like, or combinations thereof. The coating may further comprise lecithin, which serves as an anti-sticking agent. Preferably, the particles are coating is an enteric coating. Enteric coatings include any barrier known in the art that is applied to oral medications, food supplements, or the like that prevents the release of the active agent before it reaches the small intestine. Enteric coatings prevent the destruction of the active agent by the acidic environment of the stomach. Typically, enteric coatings are stable at very acidic pH, such as in the stomach, and break down rapidly in mildly acidic or higher pH, such as in the small intestine. Suitable enteric coatings include, but are not limited to a shellac, such as MARCOAT^{®} 125 from Emerson Resources, Inc. (Norristown, PA), methacrylic acid copolymers and their derivatives, such as EUDRAGIT^{®} from Degussa, cellulose acetate, such as Cellulose Acetate Phthalate, NF ("CAP") from Eastman Chemical Co. (Kingston, TN), styrol maleic acid copolymers, polymethacrylic acid/acrylic acid copolymer, hydroxylpropyl methyl cellulose phthalate, polyvinyl acetate phthalate, hydroxyethyl ethyl cellulose phthalate, hydroxypropyl methyl cellulose acetate succinate, cellulose acetate tetrahydrophthalate, acrylic resin, trimellitate, zein, calcium alginate, fatty acids, fats, and combinations thereof, among others.

The enteric coating is formed or deposited on the exterior surfaces of the particles in a manner in which the enteric coating substantially encapsulates the particles. "Encapsulation" or equivalent language means the enteric coating formed on the particles covers essentially the entire outer surface of the particles. The extent of encapsulation by the enteric coating must be sufficient such that the active agents are not overly exposed immediately to the gastric juices of the stomach upon consumption so that the glucosinolate and beta-thioglucosidase particles are released prior to the small intestine.

Preferably, the coating is of uniform thickness to provide substantially a uniform rate of release among the particles. Generally, "uniform thickness" is intended to mean that the thickness of the coating does not vary more than about 50 percent, and preferably not more than about 25 percent. The coating is generally applied to provide a coating of about 10 to about 40 percent based on the weight of the dried particles.

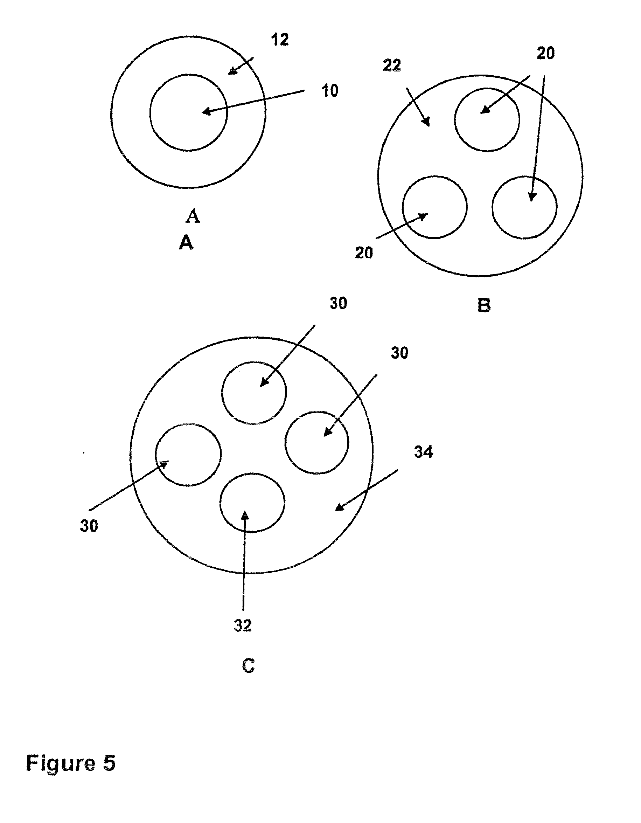

Non-limiting examples of coated particles are illustrated in FIG. 5. The particles may be coated so that only a single particle 10, such as comprising either glucosinolate or beta-thioglucosidase, is coated with a coating 12, as shown in FIG. 5A, or so that a plurality of particles 20 of a single type, such as comprising either glucosinolate or beta-thioglucosidase, are coated together with a coating 22, as shown in FIG. 5B. It may also be desired that glucosinolate particles 30 and beta-thioglucosidase particles 32 be combined and coated together with a coating 34, as shown in FIG. 5C.

In one embodiment, the particles are coated with an enteric coating composition by suspending the particles in a fluid bed and spraying them with the coating composition, followed by drying, and recovering the coated particles. Such fluid bed coating systems can include top spray systems, bottom spray systems, tangential (rotor) spray systems, and the like. Suitable multi-purpose fluid bed processors also are generally known for particle coating applications that enable different types of spray nozzle inserts to be readily installed in a common spray system, so that the same processor can be operated to apply a coating variously as a top spray, Wurster spray, or tangential spray. A coating system comprising a rotary drum coater also could be used. Of course, other coating or application systems, including coextrusion and film processing, could also be used. These coating processes can be run continuously or batch style. Suitable equipment for applying the coating on particles with these types of spray systems are commercially available. For example, suitable top spray and bottom spray fluid bed coaters available from Glatt Air Techniques, Inc. (Ramsey, NJ), can be used or readily adapted for use in applying the coating of the particles. If desired, additional coating layers may be applied which may, if desired, contain different combinations of release-modifying coatings or ingredients.

Upon ingestion or placement in a very low pH environment (such as in gastric juice in the stomach), the enteric coating does not readily dissolve. Instead, the enteric coating dissolves at a higher pH, such as in the small intestine. As the enteric coating begins to dissolve, the glucosinolates and beta-thioglucosidase enzymes are released so that beta-thioglucosidases catalyze the conversion of glucosinolate to isothiocyanates.

After coating, the particles are dried by any means known in the art, such as by fluidizing, drum drying, tray drying, vacuum drying, conventional oven, or the like, although fluidizing is preferred. Generally, the particles should contain less than about 5 percent moisture.

The enteric-coated particles of the invention may be formulated into a variety of compositions, such as compressed tablets, pills, capsules, lozenges, pharmaceuticals, or the like. In one particular aspect, the enteric-coated particles of the invention can be further processed into tablets by combining the particles with conventional tablet binders, such as starch, gelatin, sugar (such as glucose, fructose, lactose, and the like), the like, or mixtures thereof. The tablet binders should be food grade or pharmaceutically-acceptable ingredients. In preparing these compositions, the proportions of the beta-thioglucosidase and glucosinolate particles are not particularly limited as long as sufficient amounts of each respective component is present in the composition to sustain their respective intended purpose. Namely, sufficient beta-thioglucosidase and glucosinolate particles should be included such that the desired conversion of glucosinolates into isothiocyanates by the beta-thioglucosidase enzymes is achieved upon release of the particles in the small intestine. Preferably, the beta-thioglucosidase particles and glucosinolate particles of the invention should be provided in a ratio of about 100:1 to about 1:100, preferably 10:1 to about 1:10. The thickness of the coating on the compositions should be sufficient to provide the desired release of both the glucosinolate and beta-thioglucosidase particles so that both components are available for reaction in the small intestine at substantially the same time. Preferably, the coating is of uniform thickness to provide substantially a uniform rate of release among the particles. Generally, "uniform thickness" is intended to mean that the thickness of the coating does not vary more than about 50 percent, and preferably not more than about 25 percent. Generally, the coating is greater than about 10 microns thick, preferably about 20 to about 40 microns.

The enteric-coated particles of the invention may also be incorporated into food products. The enteric-coated glucosinolate and beta-thioglucosidase particles may be incorporated directly into food products or may be further processed, as desired, before incorporation into food products or pharmaceuticals. Food products into which the enteric-coated glucosinolate and beta-thioglucosidase particles may be incorporated include food supplements, nutrition bars, cereals, biscuits, drinks, shakes, pills, tablets, powdered beverage mixes, and the like, as well as mixtures thereof. Supplements include dietary supplements, nutritional supplements, herbal supplements, and the like, as well as mixtures thereof. Preferably, food products containing the enteric-coated beta-thioglucosidase and glucosinolate particles of the invention contain a ratio of beta-thioglucosidase to glucosinolate particles of about 100:1 to about 1:100, preferably 10:1 to about 1:10. Generally, the food products or pharmaceuticals contain about 1 to about 100 mg of glucosinolate particles, preferably glucoraphanin, and about 1 to about 100 mg of beta-thioglucosidase particles, preferably myrosinase, per single serving of the food product or pharmaceutical.

In another aspect, an effective amount of uncoated glucosinolate and beta-thioglucosidase particles can be filled into capsules, such as gelatin capsules, plant-based capsules, or the like. The uncoated beta-thioglucosidase particles and uncoated glucosinolate particles can be mixed together, preferably in a ratio of about 1:100 to about 100:1, more preferably about 1:10 to about 10:1, although other ratios may be used if desired. Optionally, the uncoated particles can be mixed with filler ingredients, such as lactose, or other medicaments, such as vitamins, minerals, or the like, before being filled into capsules. The capsules, tablets, or caplets are then coated with an enteric coating by any conventional method, such as by dip coating, spray coating, brush coating, pan coating, fluidized bed coating, enrobing, or the like. Generally, the coating is greater than about 10 microns thick, preferably about 20 to about 40 microns. Generally, the coating is applied to provide a coating of about 5 to about 15 percent, preferably about 8 to about 12 weight percent. Again, it is preferable that the coating be of uniform thickness to provide substantially a uniform rate of release among the particles.

The following examples are intended to illustrate the invention and not to limit it. Unless noted otherwise, percentages and ratios throughout the specification are by weight.

### EXAMPLES

### Example 1: Enteric-Coated Glucosinolate and Myrosinase Particles

### A) Creation of Glucosinolate and Beta-thioglucosidase Particles

The production of glucosinolate and beta-thiogiucosidase particles can generally be carried out using four sequential steps. The first of these steps is the formulation and creation of a compactable mixture. Following this step, the mixture is fed into a granulator/extruder to create a compact extrudate. The resulting extrudate is then fed into the spheronization equipment to form glucosinolate or beta-thioglucosidase particles. The particles are recovered and then dried in a fluidized bed.

### Myrosinase Particles

Refined lactose from Davisco Foods International, Inc. (Eden Prairie, Minnesota) at 35 percent and Microcrystalline Cellulose GP-1030 from FMC BioPolymer (Philadelphia, PA) at 45 percent were combined with white mustard seed extract (Palmieri et al., J. Agric. Food Chem., 34: 138-140 (1986)) at 20 percent. The white mustard seed extract contains myrosinase, with a specific activity of 300 nmol/min/mg protein. The dry ingredients were first tumbled together in a closed plastic weighing vessel and then placed in a Hobart stand mixer (model N-50, manufactured by Hobart Manufacturing Company (Troy, OH)) and mixed until homogenous. Under continuous mixing, the homogenously combined dry mixture was then slowly wetted with cold water (about 20°C) using a disposable 5 ml pipette to a wet mass percentage of 36 percent. After water addition, the Hobart mixer speed setting was increased for twenty seconds to form a wet mass.

### Glucosinolate Particles

Refined lactose at 28.5 percent and Microcrystalline Cellulose GP-1030 at 52.0 percent were combined with glucosinolate (produced by the method described in U.S. Application Serial No. 11/199,752 to West et al., which is incorporated herein by reference in its entirety) at a weight percentage of 19.5 percent with the same method as described above. The mixture was wetted with cold water to a wet mass percentage of 36 percent. After water addition, the Hobart mixer speed was increased to "2" for twenty seconds to form a wet mass.

The glucosinolate and myrosinase mixtures were then separately processed in a granulator, a MG-55 Single-Screw Multi Granulator distributed by LCl Corporation (Charlotte, N.C.). The mixtures were slowly fed into a hopper and separately granulated/extruded at a screw rotational speed of 50 rpm though a 0.8 mm/0.8 T dome die.

The glucosinolate and myrosinase extrudates produced in the granulator were then separately placed in the spheronization equipment, a Marumerizer (model QJ-230T distributed by LCl Corporation). Once the 2.0 mm friction disk was up to 1500 rpm, the extrudates were separately fed through the hopper/lid and spheronized for three minutes or until uniform particles were created. The resulting wet particles were then collected. A Mini-Glatt (distributed by Glatt Air Techniques, Inc. (Ramsey, N.J.)), was set up for drying and warmed to 40°C. Once warmed, the wet particles were separately placed in the chamber and fluidized for approximately fifty-five minutes until dry. The glucosinolate and myrosinase particles were then stored in separate opaque containers at 4°C.

### B) Coating of Particles with Shellac

The Mini-Glatt was initiated at a starting temperature determined for each sample. The particles were placed in the chamber and allowed to fluidize. A coating material of MARCOAT^{®} 125, a solution of shellac in an ethanol/water solvent system produced by Emerson Resources, Inc. (Norristown, PA), was mixed and then measured to provide a 30 percent coating by solids weight percentage. The coating material was then delivered into the Mini-Glatt and onto the fluidized particles using a Flocon 1003 pump manufactured by Roto-Consulta (Lucerne, Switzerland). The Mini-Glatt settings for coating the myrosinase particles and glucosinolate particles were as detailed in Table 1 below.

**Table 1:**

| **Mini-Glatt Settings** | **Myrosinase Particles** | **Glucosinolate Particles** |
|---|---|---|
| Inlet air pressure | 0.7 bar at 24°C | 0.7 bar at 40°C |
| Spray air pressure | 1.0 bar | 1.0 bar |
| Pump rate | 0.6 ml/min (setting 5) | 0.48 ml/min (setting 4) |

The coating solutions were slowly applied to the particles until a coating of 30 percent was achieved. The coated particles were additionally fluidized following coating to assure a dried final product. The coated particles were then stored in opaque containers at 4°C.

### C) Incubation of Enteric-Coated Particles in Simulated Gastrointestinal Fluids

The extent of dissolution in the gastrointestinal tract was estimated using simulated biological fluids. Simulated gastric juice (pH 1.2) and simulated intestinal fluid (pH 6.8) test solutions were prepared according to USP Edition 29, p. 3171, which is hereby incorporated by reference. To simulate gastric and intestinal digestion, 50 mg of encapsulate produced in Part B of this example was weighed into 15 ml polypropylene centrifuge tubes. Then 10 ml of simulated gastric or intestinal fluid solution warmed to 37°C was added and the tubes capped. The tubes were rotated end-over-end at 20 rpm and at 37°C for one hour and then drained through a glass microfiber filter (VWR grade 691 (West Chester, PA)) to remove the undissolved material.

The filtrate was collected. For myrosinase-containing encapsulates, myrosinase activity of filtrates was analyzed by the direct spectrophotometric assay (Palmieri et al., Analytical Biochemistry, 123: 320-324 (1982), which is incorporated herein by reference). For glucosinolate-containing encapsulates, analysis of the filtrates was performed by HPLC as described in West et al., J. Chromatography A, 966: 227-232 (2002), which is incorporated herein by reference.

Duplicate batches of shellac-coated myrosinase particles and uncoated myrosinase particles (i.e., controls) were examined. No activity for the uncoated myrosinase was detected after one hour incubation in simulated gastric juice. However, shellac-coated myrosinase retained 56-85 percent of their initial activity under the same conditions. In both experiments, shellac-coated myrosinase dissolved to release 95-103 percent of the initial activity after one hour incubation in simulated intestinal fluid.

Uncoated glucosinolate particles dissolved completely and rapidly in simulated gastric and intestinal fluids, whereas shellac-coated glucosinolate particles still retained 50-59 percent of the glucosinolates after immersion for one hour in simulated gastric fluid at body temperature. Importantly, the shellac-coated glucosinolate particles released 100 percent of the glucosinolates in simulated intestinal fluid.

### Example 2: Enteric-Coated Capsules Containing Uncoated Glucosinolates and Myrosinase

Gelatin capsules from Wonder Laboratories (White House, TN) were coated with either EUDRAGIT^{®} coatings from Degussa or Cellulose Acetate Phthalate ("CAP") from Eastman Chemical Co. (Kingston, T.N.) using a ProCoater from Torpac (Fairfield, N.J.). EUDRAGIT^{®} and CAP coatings are resistant to stomach acid.
Generally, the coatings are applied to provide a coating of about 8 to about 12 percent. Glucosinolates and beta-thioglucosidases were stable, as measured by HPLC, during the coating process. The coated capsules were tested according to the Torpac method (Fairfield, NJ) and remained stable in 0.1 N HCl for over 2 hours at 37°C but fully dissolved in pH 6.8 phosphate buffer in 30 minutes at 37°C.

While the invention has been described in terms of preferred embodiments, those skilled in the art will recognize that the invention can be practiced with modification within the spirit and scope of the appended claims.

## Claims

1. An enteric-coated chemoprotectant precursor composition comprising:
(1) about 0.5 to about 50 weight percent glucosinolate;
(2) about 0.5 to about 50 weight percent beta-thioglucosidase; and
(3) about 1 to about 99 weight percent composition comprising coating excipient and optionally diluent,
wherein the chemoprotectant precursor composition is encapsulated with enteric coating.

2. The enteric-coated chemoprotectant precursor composition of Claim 1, wherein the beta-thioglucosidase particles are myrosinase particles and the glucosinolate particles are glucoraphanin particles.

3. The enteric-coated chemoprotectant precursor composition of Claim 1 or 2, further comprising enzyme activator selected from the group consisting of ascorbic acid and its derivatives, or a combination thereof.

4. The enteric-coated chemoprotectant precursor composition of Claim 3, wherein the enzyme activator is present in an amount of about 0.001 to about 10 weight percent.

5. The enteric-coated chemoprotectant precursor composition of any one of Claims 1 to 4, wherein the coating excipient is selected from the group consisting of microcrystalline cellulose and its derivatives, oat fiber, carboxy methyl cellulose, or a combination thereof.

6. The enteric-coated chemoprotectant precursor composition of any one of Claims 1 to 5, wherein the diluent is selected from the group consisting of lactose, starch, dextrin, water, glycerol, sorbitol, propylene glycol, or a combination thereof.

7. The enteric-coated chemoprotectant precursor composition of any one of Claims 1 to 6, wherein the enteric coating is selected from the group consisting of shellac, calcium alginate, zein, fatty acids, fats, or a combination thereof.

8. A food product or a pharmaceutical composition comprising an effective amount of the enteric-coated chemoprotectant precursor composition of any one of Claims 1 to 7.

9. The food product of Claim 8, wherein the glucosinolate is glucoraphanin and the beta-thioglucosinolate is myrosinase.

10. The pharmaceutical composition of Claim 8, wherein the glucosinolate is glucoraphanin and the beta-thioglucosinolate is myrosinase.

11. An enteric-coated capsule comprising a chemoprotectant precursor mixture, the chemoprotectant mixture comprising uncoated beta-thioglucosidase particles and uncoated glucosinolate particles in a ratio of about 1:100 to about 100:1, wherein the mixture is contained within an enteric-coated capsule.

12. The enteric-coated capsule of Claim 11, wherein the beta-thioglucosidase is myrosinase and the glucosinolate is glucoraphanin.

13. The enteric-coated capsule of Claim 11 or 12, wherein the chemoprotectant precursor mixture further comprises about 0.001 to about 10 weight percent enzyme activator selected from the group consisting of ascorbic acid and its derivatives, or a combination thereof.

14. The enteric-coated capsule of Claim 11, wherein the chemoprotectant mixture comprises uncoated beta-thioglucosidase particles and uncoated glucosinolate particles in a ratio of about 1:10 to about 10:1.

15. The enteric-coated capsule of any one of Claims 11 to 14, wherein the enteric coating is selected from the group consisting of shellac, zein, fatty acids, fats, or a combination thereof.

16. The enteric-coated chemoprotectant precursor composition according to any one of Claims 1 to 7, the food product of Claim 8 or 9, the pharmaceutical composition of Claims 8 or 9 or the enteric-coated capsule of any one of Claims 11 to 15 for use in a method of converting glucosinolate to isothiocyanate in the small intenstine wherein beta-thioglucosidase and glucosinolate are released from the enteric-coated beta-thioglucosidase particles and enteric-coated glucosinolate particles, respectively, in the small intestine where beta-thioglucosidase converts glucosinolate to isothiocyanate.

17. The composition, food product, pharmaceutical composition of the enteric-coated capsule according to Claim 16, wherein the beta-thioglucosidase particles and glucosinolate particles are formed by spheronization.
